# EUROPEAN PATENT APPLICATION

(11) **EP 1 748 072 A1**
(43) Date of publication of application: **31.01.2007**
(21) Application number: 06015618.9
(22) Date of filing: 26.07.2006
(51) Int. Cl.: C12N 15/10, B03C 1/00

(54) **Hydroxysilane functionalized magnetic particles and nucleic acid separation method**

(30) Priority: 28.07.2005 US 703386 P
(71) Applicant: Polysciences, Inc., Warrington, Pennsylvania 18976 (US)
(72) Inventor: Templer, David A., Dresher, Pennsylvania 19025 (US)
(74) Representative: Katzameyer, Michael

(57) **Abstract**

A method for obtaining nucleic acids from a sample, includes: providing the sample including nucleic acids and other components; providing paramagnetic particles including a metal oxide core and a hydroxysilane (preferably a hydroxyalkyltrialkoxysilane) coating; contacting the sample with the paramagnetic particles under binding conditions such that the nucleic acids bind to the paramagnetic particles to provide loaded particles; separating the loaded particles from the other components of the sample; and releasing the nucleic acids from the loaded particle under eluting conditions to obtain the nucleic acids. A paramagnetic particle including a metal oxide core and a hydroxysilane (preferably a hydroxyalkyltrialkoxysilane) coating, and a kit including the particle are also described.

## Description

### BACKGROUND OF THE INVENTION

### 1. FIELD OF INVENTION

This invention relates to magnetic particles functionalized with a hydroxysilane and to methods comprising the use of such particles to isolate nucleic acids.

### 2. DESCRIPTION OF RELATED ART

The isolation of nucleic acids is a fundamental step in many areas of molecular biology research. A vast number of patent publications relate to nucleic acid isolation.

For example, U.S. Patent No. 6,589,799 to Coyne et al. discloses a method for producing a derivatized aldehydic support, wherein surface hydroxyl groups on the support are reacted with aldehydic alkoxy silanes to provide a derivatized aldehydic support useful for immobilizing biomolecules, including nucleic acids and proteins. Disclosed support materials include glasses, agarose, silica, alumina, glass-coated ELISA plates, resin, nickel, aluminum, zinc and paramagnetic iron. No examples of nucleic acid isolation are given.

U.S. Patent No. 4,695,392 to Whitehead et al. discloses magnetically responsive particles comprising a metal oxide core surrounded by a silane coating to which a wide variety of organic and/or biological molecules may be coupled. The patent states that nucleic acids can be isolated using these particles, but provides no examples of nucleic acid isolation, nor any guidance regarding the same. See also U.S. Patents Nos. 4,554,088, 4,628,037 and 4,695,393, all to Whitehead et al.

U.S. Patent No. 5,759,820 to Homes et al. discloses a cDNA production method, wherein mRNA is isolated on an insoluble support comprising magnetic particles that are monodisperse polymer particles comprising superparamagnetic iron oxide, a coating to reduce non-specific binding and a substituent for attaching an oligonucleotide. Probes for the mRNA are attached to the particles by chemical bonding or affinity binding. Functional groups on the particles, such as hydroxyl, carboxyl, aldehyde or amino groups, facilitate attachment of the probes to the particles.

U.S. Patent No. 6,534,262 to McKernan et al. discloses a method of isolating target nucleic acid molecules from a solution comprising a mixture of different size nucleic acid molecules, in the presence or absence of other biomolecules, by adjusting the adsorption of a particular species of nucleic acid molecule to the functional group-coated surface of magnetically responsive paramagnetic particles. Adsorption is adjusted by manipulating the ionic strength and/or precipitating agent concentration of the solution to selectively precipitate, and reversibly adsorb, the target species of nucleic acid molecule, characterized by a particular molecular size, to paramagnetic particles, the surfaces of which act as a bioaffinity adsorbent for the nucleic acids. Suitable functional groups for the particle surface include amino-coated, carboxyl-coated and encapsulated carboxyl group-coated paramagnetic particles. See also U.S. Patent Application Publication No. US 2002/0106686 A1 to McKernan.

U.S. Patent No. 5,898,071 to Hawkins discloses a method of separating polynucleotides from a solution containing polynucleotides by reversibly and non-specifically binding the polynucleotides to a solid surface, such as a magnetic particle, having a functional group-coated surface. The salt and polyalkylene glycol concentration of the solution is adjusted to levels which result in polynucleotide binding to the magnetic particles. The magnetic particles with bound polynucleotides are separated from the solution and the polynucleotides are eluted from the magnetic particles. Suitable functional groups coated on the surface of the particles include carboxylic acid groups, thiol groups and streptavidin.

Despite the foregoing developments, there is still room in the art for additional nucleic acid separation methods.

All references cited herein are incorporated herein by reference in their entireties.

### BRIEF SUMMARY OF THE INVENTION

Accordingly, a first aspect of the invention comprises a method for obtaining nucleic acids from a sample, said method comprising: (a) providing the sample comprising nucleic acids and other components; (b) providing paramagnetic particles comprising a metal oxide core and a hydroxysilane coating; (c) contacting the sample with the paramagnetic particles under binding conditions such that the nucleic acids bind to the paramagnetic particles to provide loaded particles; separating the loaded particles from the other components of the sample; and releasing the nucleic acids from the loaded particles under eluting conditions to obtain the nucleic acids.

A second aspect of the invention comprises a paramagnetic particle comprising a metal oxide core and a hydroxysilane coating.

A third aspect of the invention comprises a kit for obtaining nucleic acids from a sample. The kit comprises the paramagnetic particles of the invention, and a binding buffer comprising a salt and an alcohol at concentrations suitable for reversibly binding the nucleic acids onto surfaces of the paramagnetic particles.

### DETAILED DESCRIPTION OF PREFERRED EMBODIMENTS OF THE INVENTION

The method of the invention provides a convenient and rapid separation of nucleic acids, such as DNA, RNA and analogs thereof, from other biomolecules, such as proteins, monosaccharides, polysaccharides, lipids and cellular components, such as cell membranes.

The method of the invention comprises a step of reversibly binding nucleic acids to paramagnetic particles whose surfaces are coated with functional groups comprising hydroxyls. In a preferred embodiment of the method, the paramagnetic particles are combined with a solution of nucleic acid, after which the salt concentration and/or the alcohol concentration of the resulting combination are adjusted to binding concentrations suitable for binding nucleic acids to the surface of the paramagnetic particles. As a result, nucleic acids are bound to the surfaces of the paramagnetic particles. Subsequently, the paramagnetic particles in the resulting combination are separated from the supernatant. The paramagnetic particles having nucleic acids bound thereto (i.e., the "loaded particles") can, optionally, be washed with a suitable wash buffer before they are contacted with a suitable elution buffer, to elute and separate the nucleic acids from the paramagnetic particles. In a final step, the paramagnetic particles are separated from the elution buffer, which contains the nucleic acids in solution. The paramagnetic particles are separated from the elution buffer by, for example, filtration or applying a magnetic field to draw down the particles.

As used herein, "paramagnetic particles" are particles which are attracted by a magnetic field. The paramagnetic particles used in the method of the present invention comprise a paramagnetic metal oxide core, which is generally surrounded by an adsorptively or covalently bound silane coat. The magnetic metal oxide core is preferably iron oxide, wherein iron is a mixture of Fe²⁺ and Fe³⁺. The preferred Fe²⁺/Fe³⁺ ratio is preferably 2/1, but can vary from about 0.5/1 to about 4/1. Paramagnetic particles comprising an iron oxide core without a silane coat can be obtained from Polysciences, Inc. of Warrington, PA (BIOMAG Superparamagnetic Iron Oxide particles) for use in preparing the paramagnetic particles of the invention. Alternatively, the uncoated core can be prepared by a method based on the teachings of U.S. Patents Nos. 4,695,392, 4,628,037, 4,554,088, 4,672,040, 4,695,393 and 4,698,302 (all to Whitehead, Josephson and/or Chagnon).

Rather than coating the metal oxide core with aminosilanes as primarily taught in those patents, however, the core is coated with a silane composition that presents free hydroxyl groups for binding nucleic acids. The term "hydroxysilanes" is used herein to denote the class of silane compositions, which present free hydroxyl groups for binding. Preferred hydroxysilanes useful to coat the particle surfaces include but are not limited to hydroxyalkyltrialkoxysilanes and hydoxyalkyldialkoxysilanes, wherein the alkyl is preferably a C1 to C3 alkyl and the alkoxy is preferably a C1 to C3 alkoxy. Most preferably, the core is coated with hydroxymethyltriethoxysilane.

Paramagnetic particles useful in the present method can be a variety of shapes, which can be regular or irregular. Preferably, the shape maximizes the surface areas of the particles. The paramagnetic particles should be of such a size that their separation from solution, for example by filtration or magnetic separation, is not difficult. In addition, the magnetic particles should not be so large that surface area is minimized or that they are not suitable for microscale operations. Suitable sizes range from about 0.1 µm mean diameter to about 100µm mean diameter. A preferred size is about 1 µm mean diameter.

The paramagnetic particles are contacted with a sample containing nucleic acids under binding conditions such that the nucleic acids bind to the paramagnetic particles to provide loaded particles. The binding is preferably non-specific binding. The expression "non-specific binding" as used herein refers to binding of different nucleic acid molecules with approximately the same affinity to the paramagnetic particles, despite differences in the nucleic acid sequence or size of the different molecules. The expression "nucleic acid" as used herein includes oligonucleotides, polynucleotides, DNA, RNA or synthetic analogs thereof.

The sample containing the nucleic acids comprises other components to be separated from the nucleic acids. Such components are not particularly limited, except it is preferred that the other components have no affinity or a reduced affinity for the paramagnetic particles. The other components include but are not limited to biomolecules other than nucleic acids (e.g., proteins, carbohydrates, etc.), inorganic compounds and organic compounds. The other components are typically (but not exclusively) derived from materials obtained from an organism along with the nucleic acids. In certain embodiments, the sample contains nucleic acids which are the reaction product of PCR amplification, a cleared lysate or an agarose solution prepared in accordance with the teachings of U.S. Patent No. 5,898,071 to Hawkins. The sample is preferably provided in the form of an aqueous solution containing the nucleic acids and the other components.

Conditions of the sample are modified to provide binding conditions and eluting conditions for the appropriate steps in the method. A "binding condition" is a condition of the sample under which binding of nucleic acids to the paramagnetic particles occurs. An "eluting condition" is a condition of the sample under which nucleic acids bound to the paramagnetic particles are released. In preferred embodiments, the concentration of a salt in the sample and/or a concentration of an alcohol in the sample is/are adjusted to provide binding conditions and eluting conditions.

Salts suitable for controlling the binding of nucleic acids to the paramagnetic particles include but are not limited to sodium chloride, lithium chloride, barium chloride, potassium chloride, calcium chloride, magnesium chloride and cesium chloride, with sodium chloride being most preferred. Other suitable salts include salts of halides other than chlorine with akali and alkaline earth metals. The wide range of salts suitable for use in the method indicates that many other salts can also be used and can be readily determined by one of ordinary skill in the art using the present disclosure as a guide. Yields of bound nucleic acid decrease if the final salt concentration is adjusted to less than about 0.1M or greater than about 0.5M. The salt concentration is preferably adjusted to about 0.15M to provide binding conditions to the sample.

Alcohols suitable for controlling the binding of nucleic acids to the paramagnetic particles include but are not limited to ethanol and polyols. The alcohol concentration is preferably adjusted to 10 vol.% to 100 vol.%, more preferably about 20 to 100 vol.%, to provide binding conditions to the sample. Where the alcohol is ethanol, the preferred concentration is 50 vol.% to 100 vol.%, and where the alcohol is a polyol, the preferred concentration is 10 vol.% to 100 vol.%.

In preferred embodiments of the invention, a sufficient quantity of a salt and a sufficient quantity of an alcohol are combined with the paramagnetic particles and the nucleic acid-containing sample to produce a final salt concentration of from about 0.5M to about 5.0M and a final alcohol concentration of from about 50 vol.% to about 100 vol.%. At appropriate concentrations of the two, nucleic acids bind to the surface of the paramagnetic particles.

After the nucleic acid is bound to the paramagnetic particles, the loaded particles are then separated from the remainder of the sample. Preferably, a magnetic field is applied to the sample to draw down the loaded particles, followed by removal of the supernatant containing the other components of the sample.

The loaded particles are optionally washed with a wash buffer before separating the nucleic acid from the loaded particles by washing with an elution buffer. A suitable wash buffer has several characteristics. First, the wash buffer must have a sufficiently high salt concentration (i.e., has a sufficiently high ionic strength) that the nucleic acid bound to the magnetic particles does not elute off of the particles, but remains bound to the particles. Suitable salt concentrations are greater than about 1.0M and are preferably about 5.0M. Second, the wash buffer is chosen so that impurities that are bound to the nucleic acid or particles are dissolved. The pH and solute composition and concentration of the wash buffer can be varied according to the type of impurities that are expected to be present. Suitable wash buffers include but are not limited to the following: (a) 0.5x5 SSC; (b) 100 mM ammonium sulfate, 400 mM Tris pH 9, 25 mM MgCl₂ and 1% bovine serum albumine (BSA); and (c) 5M NaCl. A preferred wash buffer comprises 25 mM Tris acetate (pH 7.8), 100 mM potassium acetate (KOAc), 10 mM magnesium acetate (Mg₂OAc), and 1 mM dithiothreital (DTT). Most preferably, the wash buffer is 1 % SSC and 70% ethanol. The loaded particles can also be washed with more than one wash buffer. The loaded particles can be washed as often as required to remove the desired impurities. However, the number of washings is preferably limited to two or three in order to minimize loss of yield of the bound nucleic acid.

Once separated from the supernatant, and following any optional washing step, the nucleic acid can be removed from the paramagnetic particles by washing with a suitable elution buffer. As a result, an elution buffer containing unbound nucleic acids and paramagnetic particles is produced. A preferred elution buffer is any aqueous solution in which the salt concentration and/or alcohol concentration is/are below the ranges required for binding of nucleic acids onto the paramagnetic particles, as discussed above. In addition, 0.1 M Tris, 0.2 M EDTA buffer (pH 7.4) and formamide (100 vol.%) solutions can be used to elute the nucleic acids. A preferred eluent is water.

Once the bound nucleic acid has been eluted, the paramagnetic particles are separated from the elution buffer that contains the eluted nucleic acid. Preferably, the paramagnetic particles are separated from the elution buffer by magnetic means, as described above. Other methods known to those skilled in the art can be used to separate the paramagnetic particles from the supernatant; for example, filtration can be used.

Yields of nucleic acid following elution are maximized when the paramagnetic particles are used in theoretical excess relative to the amount of nucleic acid in the sample. For example, if 20 µg of DNA is theoretically in the sample, more than 20 µg of paramagnetic particles are preferably used to separate the DNA from other components in the sample. In certain embodiments, the weight of particles used is 10, 20, 50, 100, or more times the theoretical weight of nucleic acid in the sample.

The nucleic acids in the sample with which the paramagnetic particles are combined can be single-stranded, double-stranded, triple-stranded, quadruple-stranded, etc. In addition, the nucleic acids in the sample can all be the same (i.e., homogeneous) or different (i.e., heterogeneous). The nucleic acids in the sample can also comprise a DNA library or partial library. The nucleic acids in the sample can also comprise molecules of various lengths. For example, nucleic acid fragments ranging from 50 bp or less to 10 Kb or more can be isolated by the method of the present invention.

Temperature does not appear to be critical in the method of separating nucleic acids of the present invention. Ambient temperature is preferred, but any temperature above the freezing point of water and below the boiling point of water can be used.

Nucleic acid fragments of all sizes bind non-specifically to magnetic particles at high ionic strength. High ionic strength refers to salt concentrations greater than 0.5M. However, smaller fragments of DNA bind with lower affinity than large DNA fragments at lower ionic strengths, for example, about 0.5M salt concentration and lower. This differential binding as a function of ionic strength can be exploited to separate a mixture of nucleic acid fragments based on size. For example, the separation method of the invention is carried out through the optional washing step as described. Fragment size discrimination can be achieved by the stepwise contacting of the loaded particles with elution buffers of increasing ionic strength to progressively elute nucleic acid fragments of increasing size.

The separation of nucleic acid fragments based on size can also be accomplished by adjusting the alcohol concentration, and/or the nature of the alcohol (ethanol vs. PEG, the molecular weight of the PEG, etc.).

A kit is also provided herein which includes paramagnetic particles comprising a metal oxide core and a hydroxysilane (preferably a hydroxyalkyltrialkoxysilane) coating; and a binding buffer comprising a salt and an alcohol at concentrations suitable for reversibly binding the nucleic acids onto surfaces of the paramagnetic particles. Preferably, the metal oxide core comprises iron oxide and the hydroxysilane coating comprises hydroxymethyltriethoxysilane.

The binding buffer comprises at least one of a suitable salt and a suitable alcohol, which is present at concentration suitable for binding nucleic acids to the surface of the paramagnetic particles. It is preferred that the salt is sodium chloride at a concentration from 0.1 to 0.5 M and the alcohol is ethanol at a concentration of 50 to 100 vol.%. In another embodiment, the salt is sodium chloride at a concentration from 0.1 to 0.5 M and the alcohol is a polyol at a concentration of 10 to 20 vol.%.

In certain embodiments, the kit further comprises an elution buffer which is capable of eluting the nucleic acids from the loaded particles. Alternatively, instead of a binding buffer and/or elution buffer, the kit can comprise the reagents for making the binding and/or elution buffer, to which a known amount of water can be added to create a binding and/or elution buffer of desired concentration.

In another embodiment, the kit further comprises a wash buffer which dissolves impurities bound to the paramagnetic particles, but does not result in elution of the nucleic acids from the loaded particles. Alternatively, instead of a wash buffer, the kit can comprise the reagents for making the wash buffer, to which a known amount of water can be added to create a wash buffer of desired concentration.

The invention will be illustrated in more detail with reference to the following Examples, but it should be understood that the present invention is not deemed to be limited thereto.

### EXAMPLES

The paramagnetic particles used in all of the Examples were BIOMAG Superparamagnetic Iron Oxide particles (available from Polysciences, Warrington, PA, as Catalog # 84200B) coated with hydroxymethyltriethoxysilane by the following method.
1. Transfer 561 grams of 8.2% BIOMAG particles ground to 1 micron size (about 46 grams) to a 1.9 liter culture flask.
2. Add 1 liter of 0.5% sodium chloride solution, and mix by shaking. Magnetically separate particles from solution and remove and discard supernatant.
3. Repeat step #2, two more times for a total of three washes.
4. Add 1 liter of methanol to the particles, and mix by shaking and/or sonication. Magnetically separate particles from solution and remove and discard supernatant.
5. Repeat step #4, two more times for a total of three washes.
6. Resuspend the particles in 500 ml of methanol and transfer to a liter nalgene bottle. Magnetically separate the particles from solution, remove and discard the supernatant. Add 500 ml of methanol to the particles.
7. Prepare O-phosphorous acid by weighing 4.7 grams and dissolving it in 200 ml of methanol.
8. Weigh out in a separate container 250 grams of hydroxymethyltriethoxy silane.
9. Add the silane and the acid to the particles simultaneously, cap the bottle and mix on a tube rotator or roller apparatus for 1 hour at room temperature.
10. Add 700 ml of glycerol to a stainless steel container setup in a heated water bath with an overhead stirrer. Heat the glycerol to 50°C and stir at a rate of 100 to 200 rpm.
11. Remove the 1 liter nalgene bottle containing the particles, silane and acid from the mixing device and transfer the contents to the stainless steel container. Increase the temperature from 50°C to about 70°C. Run water through the reflux condenser. Maintain the heat at 65 to 70°C for 1.5 to 2 hours.
12. After 2 hours, remove the reflux condenser, continue to heat and stir the product while allowing the solution to boil down to its original volume. This will take approximately 1 to 2 hours depending on the hot plate and heat setting.
13. Shut down heat and continue to stir until room temp. If leaving to stir overnight reseal the container to prevent evaporation of solvents.
14. Wash the product 6 times with 1.5 liters of 0.5% NaCl solution, using sedimentation and/or magnetic separation to pellet the particles. Remove and discard the supernatants with each wash.
15. Resuspend the product to about 50 mg/ml. Sample the product (3 x 1 ml aliquots) to determine the particle concentration. Adjust the volume if necessary.

Prior to use, the particles were prewashed two times in 0.5 M EDTA, pH 8.0, 0.02% sodium azide. (BIOMAG Prep Buffer, available from Polysciences, Inc. of Warrington, PA).

Example 1

Plant Genomic DNA Purification

85 mg of fresh plant leaves were rinsed with deionized water and blotted dry. The leaves were frozen with dry ice and ground in 500 µl plant lysis extraction buffer (0.1 M Tris, 0.1 M EDTA, 0.25 M NaCl, pH 8.0). The resulting lysate was transferred to a microcentrifuge tube. 50 µl of 10% lauroylsarcosine were added to a final concentration of 1% and 2.75 µl of 20mg/ml Proteinase K were added to a final concentration of 100 µg/ml, mixing well without vortexing. Lysis was permitted to proceed for about one hour at 55 °C, followed by centrifugation for 5 minutes at 10-12,000 x g to remove plant debris. The supernatant was transferred with gentle mixing to a microcentrifuge tube containing 50 µl of the prewashed hydroxymethyltriethoxysilane-coated BIOMAG particles. NaCl (60 µl of 5M NaCl) was then added with gentle mixing. The resulting mixture was divided into two tubes.

1.5 ml of 100% ethanol were added to each tube with gentle mixing and incubating at room temperature for 10 minutes. The tube was then placed on a magnetic separator until the supernatant cleared. The cleared supernatant was aspirated from the tube using a pipette and discarded. Contaminating proteins were removed with two 500 µl washes of Wash Buffer A (70% ethanol, 1% sodium lauryl sarcosine), wherein the sample was mixed by inversion without vortexing. After successive magnetic separations, all supernatants were aspirated and discarded. The second set of washes was with 500 µl of 70% ethanol to remove any remaining salt. The sample was gently resuspended by inverting the tube several times and then placed on a magnetic separator until the supernatant cleared. The cleared supernatant was aspirated and discarded. This wash was repeated one time, followed by air drying the particles at 15-30°C for 5 minutes. The tube was removed from the magnet and the particles were resuspended in 50 µl of elution buffer (10 mM Tris-HCl, 0.2% sodium azide, pH 7.4). The plant DNA eluted after the sample was incubated at 80°C for about 2 minutes. This step was repeated and the DNA elutes were pooled together. Performing this entire purification procedure twice yielded from each run approximately 40-60 µg of plant DNA isolated and ready for use in PCR, labeling, sequencing and cloning.

Example 2

Plasmid DNA Purification

1 ml of an overnight culture was transferred into a microcentrifuge tube and spun for 5 minutes at 5000 x g to pellet the bacterial cells. The supernatant was aspirated and discarded, and the pellet was air dried for 2 minutes. The dried pellet was resuspended in 30 µl of Solution I (50 mM glucose, 25 mM Tris, 10 mM EDTA and 0.02% sodium azide) and then treated with 10 µl of RNase A to inhibit any ribonucleases. Next, the suspension of bacterial cells was lysed for 5 minutes at room temperature in 60 µl of Solution II (0.2 N sodium hydroxide and 1% SDS). Then 45 µl of Solution III (3.0 M potassium acetate, 0.02% sodium azide) was added to the sample with gentle mixing. Solution III precipitated chromosomal DNA, denatured proteins, cellular debris, and SDS, which were then removed from the sample. The sample was then centrifuged for 5 minutes at 12,000-14,000 x g at room temperature. 100 µl of the supernatant containing the DNA were then transferred to a new tube. To the DNA were added 10 µl of the prewashed hydroxymethyltriethoxysilane-coated BIOMAG particles at a concentration of 20 mg/ml with mixing by gentle inversion. Binding Solution (11 µl of 5M NaCl) was added and mixed by inversion. 300 µl of 100% ethanol were added and mixed gently without vortexing. The tube was placed on a magnetic separator and when the supernatant cleared, it was aspirated and discarded. The DNA bound particles were washed two times in 500 µl of 70% ethanol using magnetic separation to remove any salt and any remaining cellular debris. The particles were allowed to air dry briefly (alternatively a cotton swab can be used to absorb any liquid remaining in the tube). The DNA was eluted from the particles after a 5 minute incubation at room temperature in 50 µl of elution buffer (10 mM Tris, 0.02% sodium azide, pH 7.4). This elution step was repeated and the two DNA supernatants were pooled together after the magnetic separation. 3.3 µg of DNA were obtained.

Example 3

Genomic DNA Purification

100 µl of freshly drawn whole blood were added to a microcentrifuge tube along with 300 µl of lysis buffer (4 M urea, 0.1 M Tris-HCl, 180 mM NaCl, 10 mM EDTA, 1% SDS, 5mM DTT, 400 µg/ml proteinase K, pH 7.5; an alternative buffer comprises 2 M urea, 2M guanidine thiocyanate, 50 mM Tris-HCl pH 7.5, 5 mM DTT, 1% n-lauryl sarcosine and 12.5 mM sodium citrate), followed by inversion mixing and incubation at 50°C for 10 minutes. 300 µl of Protein Precipitation Solution (10 M ammonium acetate) were added, followed by a 2-3 minute incubation on ice to precipitate proteins in the sample. The sample was centrifuged at 12,000 x g for 5 minutes, and the clear supernatant was transferred to a clean 1.5 ml microcentrifuge tube. 50 µl of the pre-washed hydroxymethyltriethoxysilane coated BIOMAG particles (20 mg/ml w/v), were added to the cleared supernatant and mixed gently by inversion. Binding solution (65 µl of 5M NaCl) was added and mixed gently. 1.8 ml of 100% ethanol were added to the sample with mixing, followed by incubation at room temperature for 10 minutes with occasional gentle mixing. The sample was placed on a magnetic separator and when the supernatant was clear, it was aspirated and discarded. Next, the DNA-bound particles were washed to remove unwanted proteins and to give a DNA preparation by resuspending the particles in 500 µl Wash Buffer A (70% ethanol, 1% sodium lauryl sarcosine). This was mixed by inversion, followed by magnetic separation of the components. After clearing, the cleared supernatant was removed and discarded. The Wash Buffer A rinse was then repeated. The DNA bound particles were washed two times in 500 µl of 70% ethanol using magnetic separation to remove any salt and any remaining cellular debris. After separation from the supernatant, the particles were allowed to air dry briefly. The dried particles were then incubated for 2 minutes at 80°C in 40 µl of eluting buffer (10 mM Tris, 0.02% sodium azide, pH 7.4). After magnetic separation, the clear supernatant containing DNA was transferred to a fresh tube. The elution step was repeated and the supernatant DNA fractions were pooled. The final yield of DNA was about 30 µg.

The results from Examples 1-3 were compared with results obtained using Solid Phase Reversible Immobilization (SPRI) technology in accordance with Example 1 of U.S. Patent No. 5,898,071 to Hawkins. This comparison is shown in Table 1.

**Table 1. DNA Yields**

| | SPRI | Examples 1-3 |
|---|---|---|
| Plant genomic DNA | 5-20 µg | 40-60 µg |
| Bacterial DNA | 5 µg | 10 µg |
| Genomic DNA | 15 µg | 30 µg |

As shown in Table 1, the typical DNA yield from the inventive method is two times that of DNA isolated from a SPRI protocol. The time constraints for using both methods are equal.

While the invention has been described in detail and with reference to specific examples thereof, it will be apparent to one skilled in the art that various changes and modifications can be made therein without departing from the spirit and scope thereof.

## Claims

1. A paramagnetic particle comprising a metal oxide core and a hydroxysilane coating.

2. The paramagnetic particle of claim 1, wherein the metal oxide core comprises iron oxide and the hydroxysilane coating comprises a hydroxyalkyltrialkoxysilane.

3. The paramagnetic particle of claim 1, wherein the metal oxide core comprises iron oxide and the hydroxysilane coating comprises hydroxymethyltriethoxysilane.

4. The paramagnetic particle of claim 1, wherein a diameter of the particle is 0.1 µm to 100µm.

5. A method for obtaining nucleic acids from a sample, said method comprising:
providing the sample comprising nucleic acids and other components;
providing paramagnetic particles according to claim 1;
contacting the sample with the paramagnetic particles under binding conditions such that the nucleic acids bind to the paramagnetic particles to provide loaded particles;
separating the loaded particles from the other components of the sample; and
releasing the nucleic acids from the loaded particles under eluting conditions to obtain the nucleic acids.

6. The method of claim 5, wherein the sample is from a plant, bacteria or human, and the other components comprise at least one member selected from the group consisting of proteins, monosaccharides, polysaccharides, lipids and other cellular components.

7. The method of claim 5, wherein the nucleic acids comprise at least one member selected from the group consisting of DNA, RNA, and analogs thereof.

8. The method of claim 5, wherein the metal oxide core comprises iron oxide and the hydroxysilane coating comprises a hydroxyalkyltrialkoxysilane.

9. The method of claim 5, wherein the metal oxide core comprises iron oxide and the hydroxysilane coating comprises hydroxymethyltriethoxysilane.

10. The method of claim 5, wherein the binding conditions comprise a first salt concentration and a first alcohol concentration, and the eluting conditions comprise a second salt concentration lower than the first salt concentration and a second alcohol concentration lower than the first alcohol concentration.

11. The method of claim 10, wherein the first salt concentration is 0.1M to 0.5M of sodium chloride, the first alcohol concentration is 50 vol.% to 100 vol.% of ethanol, the second salt concentration is less than 0.5 M sodium chloride and the second alcohol concentration is less than 50 vol.% ethanol.

12. The method of claim 11, wherein the metal oxide core comprises iron oxide and the hydroxysilane coating comprises a hydroxyalkyltrialkoxysilane.

13. The method of claim 11, wherein the metal oxide core comprises iron oxide and the hydroxysilane coating comprises hydroxymethyltriethoxysilane.

14. The method of claim 5, wherein the loaded particles are separated from the other components of the sample by application of a magnetic field to the loaded particles.

15. A kit for performing the method of claim 5, said kit comprising:
the paramagnetic particles; and
a binding buffer comprising a salt and an alcohol at concentrations suitable for reversibly binding the nucleic acids onto surfaces of the paramagnetic particles.

16. The kit of claim 15, wherein the metal oxide core comprises iron oxide and the hydroxysilane coating comprises a hydroxyalkyltrialkoxysilane.

17. The kit of claim 15, wherein the metal oxide core comprises iron oxide and the hydroxysilane coating comprises hydroxymethyltriethoxysilane.

18. The kit of claim 15, wherein a mean diameter of the paramagnetic particles is 0.1 µm to 100µm.

19. The kit of claim 15, wherein the salt is sodium chloride at a concentration from 0.1M to 0.5M and the alcohol is ethanol at a concentration of 50 vol.% to 100 vol.%.

20. The kit of claim 15, further comprising an elution buffer.
